# EUROPEAN PATENT APPLICATION

(11) **EP 2 708 208 A2**
(43) Date of publication of application: **19.03.2014**
(21) Application number: 13275215.5
(22) Date of filing: 13.09.2013
(51) Int. Cl.: A61F 2/07, A61F 2/915

(54) **Endoluminal prosthesis**

(30) Priority: 14.09.2012 US 201213618356
(71) Applicant: Cook Medical Technologies LLC, Bloomington, IN 47404 (US)
(72) Inventor: Haselby, Kenneth Aaron, Battle Ground, IN Indiana 47920-9777 (US); Milner, Keith, West Lafayette, IN Indiana 47906 (US); Mishra, Shruti, Saint Louis, MO Missouri 63110 (US); Sherman, Sara Marie, Lafayette, IN Indiana 47905 (US)
(74) Representative: Jehan, Robert

(57) **Abstract**

An endoluminal prosthesis (100) for placement within a body vessel may include a tubular support structure (110) including a proximal end segment, a distal end segment, an intermediate segment positioned between the proximal end segment and the distal end segment, a lumen extending longitudinally within the support structure (110), a luminal surface, and an abluminal surface opposite the luminal surface. The prosthesis (100) may include a first layer of nonwoven electrospun fibres positioned on the luminal surface of the support structure. The prosthesis may include a second layer of nonwoven electrospun fibres positioned on the abluminal surface of the support structure (110). At least one of the proximal end segment or the distal end segment of the support structure (110) may be encapsulated within a covering including the first layer of nonwoven electrospun fibres and the second layer of nonwoven electrospun fibres. The intermediate segment of the support structure (110) may be unencapsulated within the covering.

## Description

### TECHNICAL FIELD

This disclosure relates to endoluminal medical devices for implantation within the human or animal body for treatment of endovascular disease. More particularly, it relates to an endoluminal prosthesis having a graft material and methods of manufacturing such an endoluminal prosthesis.

### BACKGROUND

Covered stents, or stent grafts, have been used to treat a variety of medical conditions, including aneurysms, occluded vessels, and restenosis. For example, an aneurysm may occur in a blood vessel in a location where, due to age, disease, or genetic predisposition, the blood vessel strength or resiliency is insufficient to enable the blood vessel wall to retain its shape as blood flows therethrough. This may result in ballooning or stretching of the blood vessel at the location having limited strength or resiliency, thereby forming an aneurysmal sac. If the aneurysm is left untreated, the blood vessel wall may continue to expand to the point where the remaining strength of the blood vessel wall is insufficient to prevent rupture. In this instance, the blood vessel may fail at the location of the aneurysm, often with fatal result.

To prevent rupture, a stent graft of a tubular construction may be introduced into the blood vessel, for example, intraluminally. Typically, the stent graft is deployed and secured in a location within the blood vessel such that the stent graft spans the aneurysmal sac. The outer surface of the stent graft, at its opposed ends, may be sealed to the interior wall of the blood vessel at locations where the blood vessel wall has not suffered a loss of strength or resiliency. Blood flow in the vessel may be channelled through the hollow interior of the stent graft, thereby reducing, or possibly eliminating, the stress on the blood vessel wall at the location of the aneurysmal sac. The graft material of the stent graft may be substantially non-porous so that blood may be prevented from leaking into the aneurysmal sac. This may reduce the risk of rupture of the blood vessel wall at the location of the aneurysmal sac while allowing blood to continue to flow through the stent graft to the downstream blood vessels without interruption.

Various materials and methods have been used to create coverings, or grafts, that may be applied to stents to form stent grafts. For example, grafts may be made using woven techniques, thin-sheet/tubing bonding, or other processes. These grafts often require manufacturing efforts and/or post-processing techniques (e.g., gluing, sewing, taping, etc.) to create a covering. Electrospinning may be used to apply a suitable biocompatible coating or covering to a medical device, such as a stent graft. Electrospinning is a process for creating a nonwoven network of fibres using an electrically charged solution that is driven from a source to a target with an electrical field. More specifically, a solution is driven from an orifice, such as a needle. A voltage is applied to the orifice resulting in a charged solution jet or stream from the orifice to the target. The jet forms a conical shape, termed a Taylor cone, as it travels from the orifice. As the distance from the orifice increases, the cone becomes stretched until the jet splits or splays into many fibres prior to reaching the target. The fibres are extremely thin, typically in the nanometre range. The collection of fibres on the target forms a thin mesh layer of fibrous material.

### SUMMARY

The present invention seeks to provide and improved prosthesis or medical device and an improved method of making such a prosthesis.

It is to be understood herein that the term prosthesis is used to denote also implantable medical devices more generally.

According to an aspect of the present invention, there is provided an endoluminal prosthesis as specified in claim 1.

According to another aspect of the present invention, there is provided an endoluminal prosthesis as specified in claim 8.

According to another aspect of the present invention, there is provided a method of preparing an endoluminal prosthesis as specified in claim 16.

The described embodiments provide an endoluminal prosthesis having a graft material and methods of manufacturing such an endoluminal prosthesis.

In one example, an endoluminal prosthesis for placement within a body vessel may include a tubular support structure. The support structure may include a proximal end segment, a distal end segment, an intermediate segment positioned between the proximal end segment and the distal end segment, a lumen extending longitudinally within the support structure, a luminal surface, and an abluminal surface opposite the luminal surface. The prosthesis may include a first layer of nonwoven electrospun fibres positioned on the luminal surface of the support structure. The prosthesis may include a second layer of nonwoven electrospun fibres positioned on the abluminal surface of the support structure. At least one of the proximal end segment or the distal end segment of the support structure may be encapsulated within a covering including the first layer of nonwoven electrospun fibres and the second layer of nonwoven electrospun fibres. The intermediate segment of the support structure may be unencapsulated within the covering.

In another example, an endoluminal prosthesis may include a support structure. The support structure may include a proximal end segment, a distal end segment, a lumen extending longitudinally within the support structure, a luminal surface, and an abluminal surface opposite the luminal surface. A first layer of nonwoven electrospun fibres may be disposed on the luminal surface of the support structure. A second layer of nonwoven electrospun fibres may be disposed on the abluminal surface of the support structure. The first layer of nonwoven electrospun fibres and the second layer of nonwoven electrospun fibres may cooperatively form a covering. The support structure may be partially encapsulated within the covering. At least a portion of the support structure may be unencapsulated within the covering.

In another example, a method for preparing an endoluminal prosthesis may include providing a support structure including a proximal end segment, a distal end segment, a lumen extending longitudinally within the support structure, a luminal surface, and an abluminal surface opposite the luminal surface. The method may include providing an electrospinning apparatus including an orifice and a mandrel. An electric potential may be applied between the orifice and the mandrel. A first layer of nonwoven fibres may be formed on an outer surface of the mandrel by electrospinning a solution from the orifice onto the outer surface of the mandrel. The first layer of nonwoven fibres may be contacted with the luminal surface of the support structure by locating the mandrel at least partially within the lumen of the support structure. A second layer of nonwoven fibres may be formed on the abluminal surface of the support structure by electrospinning the solution from the orifice onto the abluminal surface of the support structure. The support structure may be partially encapsulated within a covering formed by the first layer of nonwoven fibres and the second layer of nonwoven fibres so that at least a portion of the support structure is unencapsulated within the covering.

Other systems, methods, features, and advantages of the invention will be, or will become, apparent to one with skill in the art upon examination of the following figures and detailed description. It is intended that all such additional systems, methods, features, and advantages be within the scope of the teachings herein and be encompassed by the following claims.

### BRIEF DESCRIPTIONS OF THE DRAWINGS

Embodiments of the present invention are described below, by way of example only, with reference to the accompanying drawings, in which:
FIG. 1 illustrates one example of an endoluminal prosthesis;
FIG. 2 illustrates one example of an electrospinning apparatus;
FIG. 3 illustrates an exemplary method step for electrospinning a solution onto an outer surface of a mandrel;
FIG. 4 illustrates one example of a layer of nonwoven electrospun fibres disposed on the outer surface of a mandrel using the method shown in FIG. 3;
FIG. 5 illustrates one example of a support structure disposed on the layer of nonwoven electrospun fibres of FIG. 4;
FIG. 6 illustrates an exemplary method step for electrospinning a solution onto an abluminal surface of the support structure of FIG. 5
FIG. 7 illustrates the support structure of FIGS. 5-6 disposed between two layers of nonwoven electrospun fibres;
FIG. 8 illustrates one example of a partially encapsulated support structure;
FIG. 9 illustrates another example of a partially encapsulated support structure; and
FIG. 10 illustrates another example of a partially encapsulated support structure.

### DETAILED DESCRIPTION OF THE PRESENTLY PREFERRED EMBODIMENTS

The present disclosure relates to an endoluminal prosthesis having a graft material and methods of manufacturing such an endoluminal prosthesis.

In the present disclosure, the term "proximal" refers to a direction that is generally closest to the heart during a medical procedure, while the term "distal" refers to a direction that is farthest from the heart during a medical procedure.

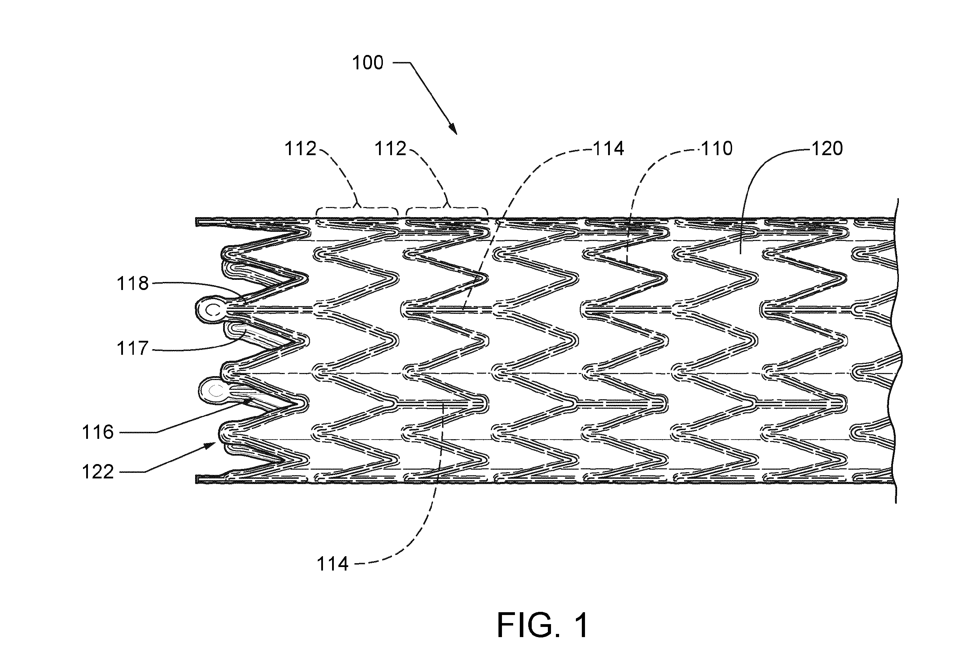

**FIG. 1** illustrates one example of an endoluminal prosthesis 100. In this example, the prosthesis 100 is a covered stent or a stent graft. The prosthesis 100 may include a support structure 110 (e.g., a stent) and a graft body 120 attached to the support structure. In one example, the support structure 110 may be partially encapsulated within the graft body 120 as shown in **FIG. 1** and further described below. The support structure may have any configuration known in the art. For example, suitable support structures may include any of those described in US Patent Nos. 8,123,794 to Flagle et al. and 8,157,857 to Case et al.*,* both of which are incorporated herein by reference. The support structure may be configured as a unitary structure or a plurality of separate structures which may collectively define the support structure. Additionally, or alternatively, the support structure may be made from a woven wire structure, a laser-cut cannula, individual interconnected rings, or another pattern or design.

In one example, the support structure 110 may include a plurality of ring structures 112 interconnected by connector segments 114 as shown in **FIG. 1****.** Each ring structure 112 may be a ring having an endless undulating pattern (e.g., a zigzag pattern). The ring structure 112 may be formed by bending a wire into the desired pattern and joining the ends of the wire, by cutting the desired pattern from a solid tube of material, or by any other suitable method. The support structure 110 may be configured as a tubular member defined by the plurality of ring structures 112. For example, the ring structures 112 may be spaced from one another longitudinally along a length of the support structure. The connector segments 114 may extend longitudinally between adjacent ring structures 112 to maintain the spacing between the ring structures.

In one example, the support structure 110 may have a substantially cylindrical shape as shown in **FIG. 1**. In other words, a transverse cross section of the support structure 110 may have a substantially circular shape. In other examples, the support structure 120 may have any other cross sectional shape including, for example, triangular, rectangular, elliptical, or any other polygonal or non-polygonal shape. A lumen 116 may extend longitudinally within the support structure 110. An inner surface or luminal surface 117 of the support structure 110 may face the lumen 116. In other words, the lumen 116 may be defined by the luminal surface 117 of the support structure 110. The support structure 110 may include an outer surface or abluminal surface 118 positioned opposite the luminal surface 117. In other words, the luminal surface 117 may be positioned inside the support structure, and the abluminal surface 118 may be positioned outside the support structure opposite the luminal surface.

The support structure 110 may add rigidity, expansion force, and/or support to the prosthesis 100. To that end, the support structure 110 may be made from one or more of numerous metals and/or alloys. For example, the support structure 110 may be made from a metallic material such as stainless steel, silver, platinum, palladium, gold, titanium, tantalum, iridium, tungsten, cobalt, chromium, cobalt-chromium alloy 1058, cobalt-based 35N alloy, nickel-based alloy 625, a molybdenum alloy, such as a molybdenum alloy including about 0.4% to about 0.8% of lanthanum oxide (La₂O₃), and a nickel-titanium alloy, such as Nitinol, or other suitable materials known in the art. In one example, the support structure 110 may include a shape-memory or superelastic material such as Nitinol. Use of a shape memory or superelastic material may enable the support structure 110 to be over expanded as further described below.

The graft body 120 may be attached to the support structure 110. The graft body 120 may be disposed on the luminal surface 117 and/or the abluminal surface 118 of the support structure 110. In one example, the graft body 120 may be disposed on both the luminal surface 117 and the abluminal surface to encapsulate the support structure 110, or a portion thereof, within the graft body as further described below. The graft body 120 may be configured as a tubular body having any suitable shape as described above with reference to the support structure 110. A lumen 122 may extend longitudinally within the graft body 120. The lumen 122 may be at least partially coextensive with the lumen 116 of the support structure 110. The lumen 122 may be configured to permit blood or other body fluids to flow through the prosthesis within the lumen 122.

The graft body 120 may be formed of any suitable graft material. In one example, the graft material may be deposited onto the support structure 110 (e.g., the luminal surface 117 and/or the abluminal surface 118) using an electrospinning process as further described below. In other examples, the graft material may be deposited onto the support structure 110 using any other suitable method including, for example, dip coating, spray coating, and melt-spinning. Many different types of biocompatible materials may be used to form the graft body 120. The biocompatible material may be substantially non-toxic in the in vivo environment of its intended use, and may be substantially unrejected by the patient's physiological system (i.e., may be non-antigenic). Examples of biocompatible materials from which a graft material may be formed include, for example, polyesters, such as polyethylene terephthalate (PET); fluorinated polymers, such as polytetrafluoroethylene (PTFE) and fibres of expanded PTFE (ePTFE), polyvinylidene fluoride (PVDF); polyurethanes; and polyolefins. Additionally, or alternatively, materials suitable for making graft materials may include polyethylene, polypropylene, polyvinyl chloride (PVC), polyaramids, polyacrylonitrile, nylon, silicone, cellulose, a biological scaffold or bioremodelable material (e.g., small intestine submucosa (SIS), commercially available from Cook Medical Incorporated, Bloomington, IN), or biodegradable materials (e.g., polylactides).

Although the discussion in this disclosure will refer to the prosthesis 100, a person having ordinary skill in the art will recognize that the devices and methods described herein may be equally applicable to a prosthesis, such as a stent or stent graft, having any other configuration. For example, the prosthesis may be configured as a bifurcated stent graft, a stent graft having branches, scallops and/or fenestrations, or a prosthesis having any other shape or features. Such devices and methods are contemplated by and within the scope of this disclosure.

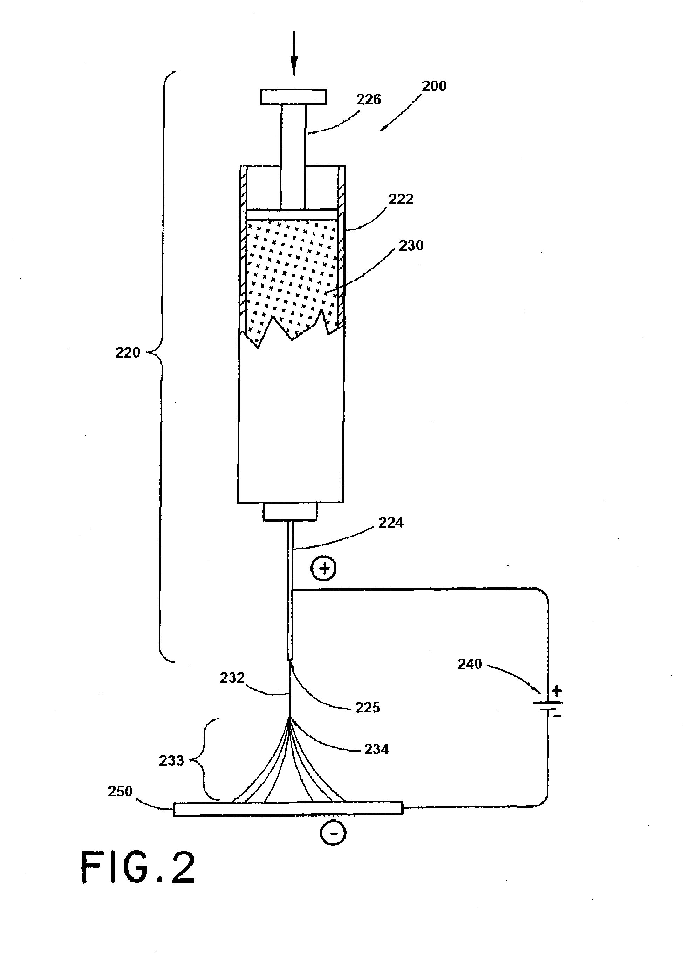

**FIG. 2** illustrates one example of an electrospinning apparatus 200 for coating an object, such as a substrate or a medical device. The electrospinning apparatus 200 may be similar to that described in U.S. Patent No. 7,799,261 to Orr et al.*,* which is incorporated herein by reference. For example, the electrospinning apparatus 200 may include a spinneret 220. The spinneret 220 may include a reservoir 222, which may be configured as a syringe-like container as shown in **FIG. 2**. The reservoir 222 may be fluidly coupled to an orifice 224 to form the spinneret 220. The orifice 224 may be configured as a needle as shown in **FIG. 2**.

A solution 230 may be loaded into the reservoir 222. Suitable solutions will be discussed in more detail below. The orifice 224 may have a distal opening 225 through which the solution 230 may be driven by a displacement system 226. The displacement system 226 may be configured as any type of controllable, variable rate fluid displacement system. For example, the fluid displacement system 226 may be configured as a plunger as shown in **FIG. 2**. Preferably, the displacement system 226 may be an automated system to provide a consistent and accurate flow of solution 230 through the orifice 224. In one example, the fluid displacement system 226 may deliver the solution 230 at a delivery rate of about 0 mL/hr to about 25 mL/hr, about 1 mL/hr to about 10 mL/hr, or about 3 mL/hr to about 7 mL/hr.

A voltage source 240 may apply an electric potential across the spinneret 220 and a target 250. In one example, the electric potential may be between about 10 kV and about 35 kV, between about 15 kV and about 30 kV, or between about 20 kV and about 25 kV. The electric potential 240 may aid the displacement system 226 in ejecting the solution 230 from the distal opening 225 of the orifice 224.

The solution may form a charged jet or stream 232 from the distal opening 225 to the target 250. The solution stream 232 may form a conical shape 233, called a Taylor cone, between the spinneret 220 and the target 250. As the solution stream 232 travels away from the opening 225, the cone 233 may begin to splay or stretch at a position 234 between the spinneret 220 and the target 250. In one example, the distance between the distal opening 225 and the target 250 may be between about 0.1 inches to about 6 inches, between about 0.5 inches to about 4 inches, or between about 1 inch to about 2 inches. Position 234 need not be substantially intermediate the distal opening 225 and the target 250, and may be located at any desired distance between the distal opening and the target. The splaying or stretching action may create a plurality of fibres that may or may not dry upon reaching the target 250, depending on the volatility of the chosen solvent. The fibres may contact the target 250 to form a coating of nonwoven fibres thereon. The coating of nonwoven fibres may be configured as a network of fibres deposited on the target 250 to collectively form a sheet of nonwoven fibres.

In one example, an electrospinning apparatus similar to the electrospinning apparatus 200 may be used to prepare an endoluminal prosthesis such as the prosthesis 100 described above. For example, an electrospinning apparatus may be used to apply a graft material to the support structure 110 to form the graft body 120 as further described below.

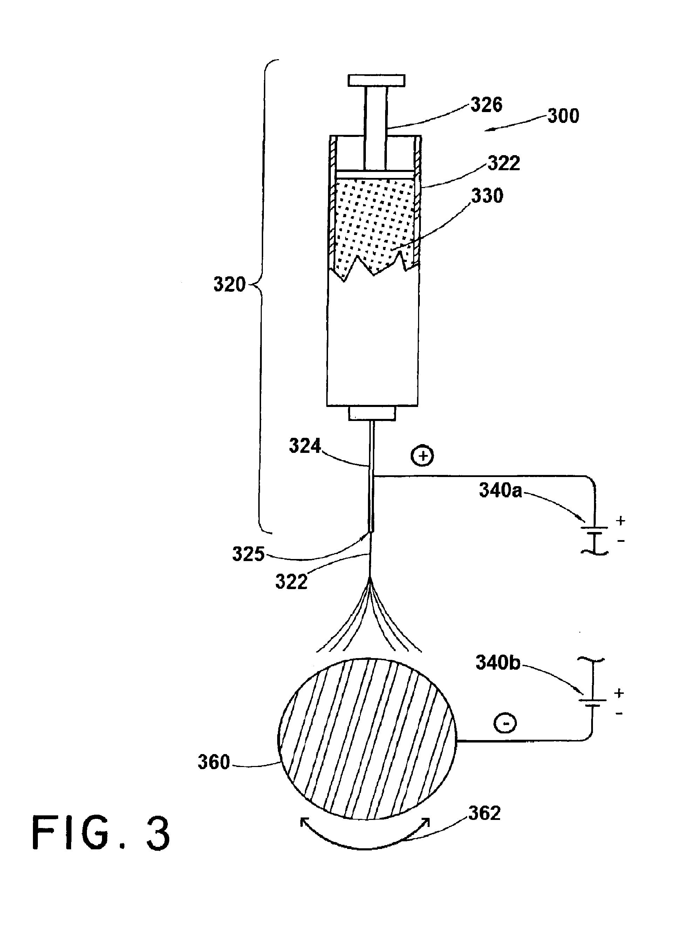

**FIG. 3** illustrates one example of an electrospinning apparatus 300, which may be used to prepare the endoluminal prosthesis 100 as further described below. For example, the electrospinning apparatus 300 may be used to electrospin a graft material (e.g., PET solution) onto a rotating mandrel to create an encapsulated supporting structure (i.e., stent) within a covering formed by the selected graft material. The electrospinning apparatus 300 may be similar to the electrospinning apparatus 200 described above. For example, the electrospinning apparatus 300 may include a spinneret 320 including a reservoir 322 that is fluidly coupled to an orifice 324. A solution 330 may be loaded into the reservoir 322 and driven by a displacement system 326 through a distal opening 325 of the orifice 324. An electric potential may be applied across the spinneret 320 and a mandrel 360. The solution may form a charged jet or stream 332 from the distal opening 325 to the mandrel 360. As the solution stream 332 travels away from the opening 325, the stream may begin to splay or stretch to create a plurality of fibres. The fibres may contact the mandrel 360 to form a coating of nonwoven fibres thereon.

In one example, a voltage source may apply an electric potential across the spinneret 320 and the mandrel 360 as described above with reference to the voltage source 140. In another example, multiple voltage sources may be used to apply the electric potential. For example, a first voltage source 340a may be electrically coupled to the spinneret 320, and a second voltage source 340b may be electrically coupled to the mandrel 360 as shown in **FIG. 3**. The first voltage source 340a may generate an electric charge on the orifice 324. In other words, the first voltage source 340a may apply an electric potential between the orifice 324 and ground. Similarly, the second voltage source 340b may generate an electric charge on the mandrel 360. In other words, the second voltage source 340b may apply an electric potential between the mandrel 360 and ground.

The electric charge on the mandrel 360 may have an opposite sign relative to the electric charge on the orifice 324. In one example, the orifice 324 may be positively charged (i.e., the sign of the electric charge may be positive), and the mandrel 360 may be negatively charged (i.e., the sign of the electric charge may be negative). In another example, the orifice 324 may be negatively charged, and the mandrel 360 may be positively charged. The magnitude of the electric charge on the orifice 324 may be the same as or different than the magnitude of the electric charge on the mandrel 360. In one example, the magnitude of the electric charge on the orifice 324 relative to ground may be between about 5 kV and about 20 kV, preferably between about 6 kV and about 7.5 kV. Additionally, or alternatively, the magnitude of the electric charge on the mandrel 360 relative to ground may be between about 5 kV and about 20 kV, preferably between about 6 kV and about 7.5 kV. The orifice 324 and the mandrel 360 may have opposing charges such that the electric potential between the orifice and the mandrel may be between about 10 kV and about 40 kV, preferably between about 12 kV and about 15 kV.

In one example, the spinneret 320 may be configured as a 3 mL plastic syringe (e.g., a NORM-JECT® syringe commercially available from Air-Tite Products Co., Virginia Beach, VA) equipped with a 23-Gauge disposable polymer-hub stainless steel needle. Additionally, or alternatively, the distance between the orifice 324 and the mandrel 360 may be between about 5 cm and about 25 cm, preferably between about 12 cm and about 15 cm. Additionally, or alternatively, the solution 330 may be extruded using a syringe pump at a substantially constant flow rate between about 0.5 mL/h and about 4 mL/h, preferably between about 0.5 mL/h and about 1.5 mL/h. Additionally, or alternatively, each of the first voltage source 340a and the second voltage source 340b may be configured as a high-voltage power supply capable of applying DC voltage up to about 20 kV.

**FIG. 3** illustrates the mandrel 360 in a cross-sectional view taken along a plane transverse to the longitudinal axis of the mandrel. In one example, the mandrel 360 may have a substantially cylindrical shape as shown in **FIG. 3**. In other examples, the mandrel may have any other suitable shape. Preferably, the mandrel 360 may be sized and shaped for placement within a lumen of a medical device (e.g., the lumen 116 of the support structure 110) as further described below. The mandrel 360 may include an outer surface extending circumferentially and longitudinally along the mandrel.

The mandrel 360 and the spinneret 320 may be movable relative to one another. Such movement may enable the coating of any portion of the outer surface of the mandrel 360. For example, the outer surface may be coated almost entirely, partially, or at discrete locations thereon. For example, the mandrel 360 may be rotatable about the longitudinal axis of the mandrel. In other words, the mandrel 360 may be configured to rotate in a direction indicated by the arrow 362. In one example, the mandrel may be configured to rotate at a speed of between about 80 rpm and about 4000 rpm, or between about 100 rpm and about 500 rpm. The rotational speed of the mandrel 360 may be adjusted to adjust the diameter of the fibres produced during electrospinning. Increasing the rotational speed of the mandrel 360 may reduce the diameter of the fibres. Decreasing the rotational speed of the mandrel 360 may increase the diameter of the fibres. Additionally, or alternatively, the mandrel 360 may be movable in a direction substantially parallel to the longitudinal axis of the mandrel. In other words, the mandrel 360 may be configured to translate (e.g., in a forward or backward longitudinal direction) relative to the spinneret 320. Additionally, or alternatively, the mandrel 360 may be movable in a direction transverse to the longitudinal axis of the mandrel. In other words, the mandrel 360 may be configured to translate (e.g., in an up, down, or sideways transverse direction) relative to the spinneret 320. Such rotation and/or translation (e.g., longitudinal or transverse translation) of the mandrel 360 relative to the spinneret 320 may enable coating of the outer surface of the mandrel, or a portion thereof, with electrospun fibres as further described below. Such a coating may be achieved by any relative motion between the mandrel 360 and the spinneret 320. For example, movement of the mandrel 360 relative to the spinneret 320 may be achieved by maintaining the spinneret in a constant position while moving the mandrel, by maintaining the mandrel in a constant position while moving the spinneret, and/or by moving the mandrel and the spinneret relative to one another. In one example, the mandrel may rotate and the spinneret may translate in a longitudinal direction relative to the mandrel.

The relative movement of the mandrel 360 with respect to the spinneret 320 may influence several properties of the resulting coating of fibres. For example, increasing the speed of the relative motion may cause a reduction in the thickness of the coating. This may be caused, for example, because a portion of the mandrel 360 may be disposed in the path of the stream 332 for a shorter period of time at increased speeds. Additionally, or alternatively, increasing the speed of the relative motion may cause the fibres to be increasingly aligned with one another. This may affect the strength, resiliency, and/or porosity of the coating. Also for example, as the distance between the spinneret 320 and the mandrel 360 is increased, the solution stream 332 may be required to travel a greater distance before reaching the mandrel. This may affect the splaying and/or drying characteristics of the solution stream 332, which may affect the properties of the resultant coating.

In any of the examples described herein, the mandrel 360 may be formed from any suitable conductive material known in the art. For example, the mandrel 360 may be formed from a metallic material such as stainless steel (e.g., electropolished stainless steel) or chrome. In another example, the mandrel 360 may be formed from a non-metallic material such as a conductive plastic material. The mandrel 360 may include a release layer disposed on the outer surface thereof to aid in removing the prosthesis 100 from the mandrel as further described below. The release layer may be formed from any material known in the art. Preferably, the release layer may be formed from a non-stick material such as, for example, PTFE, sodium bicarbonate, a silicone lubricant, or any other biocompatible lubricant.

To prepare the prosthesis 100, a layer of nonwoven fibres may be formed on the outer surface of the mandrel 360 by electrospinning the solution 330 from the orifice 324 onto the outer surface of the mandrel. In one example, the mandrel 360 may be moved rotationally about the longitudinal axis thereof. The solution 330 may be discharged from the orifice 324 and attracted to the mandrel 360 by the electrical potential applied between the orifice and the mandrel as described above. The rotation of the mandrel 360 may cause the resultant coating of nonwoven fibres to be distributed about the circumference of the mandrel. Additionally, or alternatively, the spinneret 320 may be translated longitudinally relative to the mandrel 360 while discharging the solution 330 from the orifice 324. The translation of the spinneret 320 may cause the resultant coating of nonwoven fibres to be distributed about the length of the mandrel. In one example, the mandrel 360 may be rotated and the spinneret 320 may be translated to form a layer of nonwoven fibres covering substantially the entire circumference of the mandrel along at least a portion of the length of the mandrel.

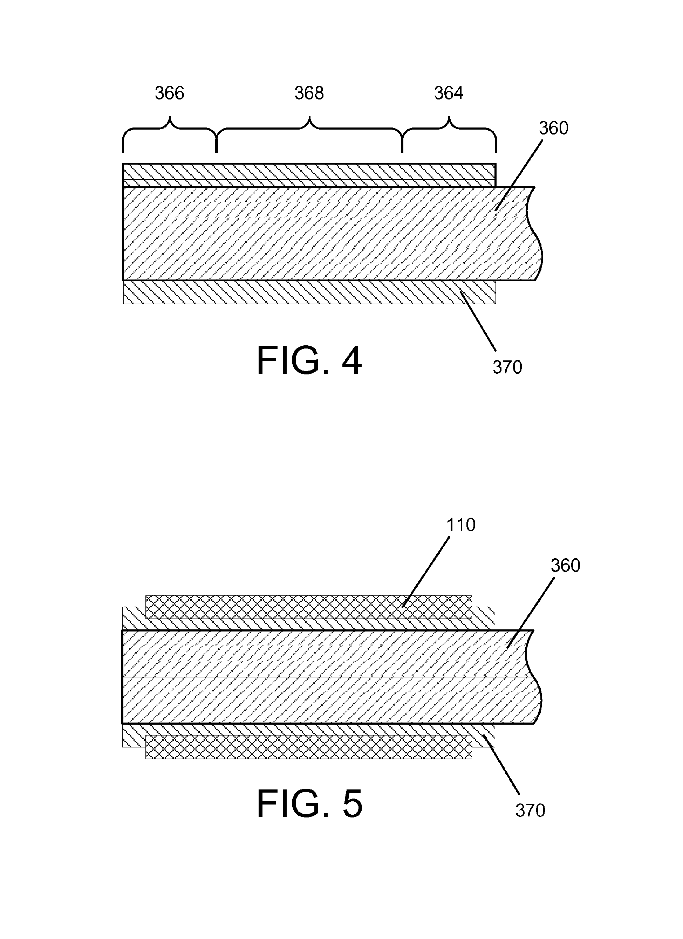

**FIG. 4** shows a longitudinal cross sectional view of an end portion of the mandrel 360 with a layer 370 of nonwoven electrospun fibres disposed on the outer surface thereof. A working length of the mandrel 360 may include a proximal end segment 364, a distal end segment 366, and an intermediate segment 368 positioned between the proximal end segment and the distal end segment. The mandrel 360 may be rotated and the spinneret 320 may be translated while the solution 330 is electrospun onto the outer surface of the mandrel as described above to distribute the electrospun fibres circumferentially and longitudinally about the outer surface of the mandrel. In one example, the solution 330 may be electrospun onto each of the proximal end segment 364, the distal end segment 366, and the intermediate segment 368 of the mandrel 360 such that the layer 370 may be disposed upon substantially the entire working length of the outer surface of the mandrel as shown in **FIG. 4**. In other words, each of the proximal end segment 364, the distal end segment 366, and the intermediate segment 368 of the mandrel 360 may be coated with the electrospun fibres. In one example, the spinneret 320 may be programmed to move longitudinally along the length of the mandrel to focus the fibres produced along substantially the entire mandrel length. The spinneret 320 may make any appropriate number of passes along the length of the mandrel 360 to achieve a coating having a desired thickness. In one example, the coating may have a thickness of between about 10 µm and about 70 µm, typically between about 15 µm and about 25 µm, preferably about 20 µm. During each pass, the spinneret 320 may move longitudinally along substantially the entire working length of the mandrel 360. The number of passes may be increased to increase the thickness of the coating of electrospun fibres or decreased to decrease the thickness of the coating of electrospun fibres. In one example, a portion of the mandrel may be uncovered by the layer 370 of nonwoven electrospun fibres to form a partially encapsulated support structure as further described below.

The support structure 110 may be placed on the mandrel 360 over the layer 370 of electrospun fibres as shown in **FIG. 5**. In this manner, the layer 370 of electrospun fibres may be positioned in contact with the luminal surface 117 of the support structure 110. In other words, the layer 370 of nonwoven fibres may be contacted with the luminal surface 117 of the support structure 110 by locating the mandrel at least partially within the lumen 116 of the support structure. In one example, the support structure 110 may have a relaxed diameter. The support structure 110 may be configured to expand to the relaxed diameter after compressing the support structure to a compressed diameter (e.g., for introducing the support structure into a body lumen in a conventional manner) and releasing the support structure in the compressed configuration. Additionally, or alternatively, the support structure 110 may be over-expandable to a diameter that is greater than the relaxed diameter (e.g., by exerting a radially outward force on the support structure in the relaxed diameter). The support structure 110 may contract toward the relaxed diameter upon releasing the support structure in the over-expanded configuration. The support structure 110 may be over-expanded for placement over the mandrel 360. In other words, the support structure 110 may be over-expanded to a first diameter that is greater than the relaxed diameter of the support structure and placed over the mandrel 360. The support structure 110 may be allowed to contract to a second diameter that is smaller than the first diameter to contact the layer 370 of nonwoven fibres on the mandrel 360. The second diameter may be greater than the relaxed diameter of the support structure 110. In other words, the support structure 110 may be over-expanded even in the second diameter configuration.

In one example, the support structure 110 may be embedded in the layer 370 of nonwoven fibres such that the layer extends into one or more openings or interstices of the support structure. In other words, at least a portion of the layer 370 may be positioned within one or more openings (e.g., formed between adjacent ring structures 112 and/or connector segments 114). This may aid in encapsulating the support structure 110 within the graft body 120 as further described below. The over-expanded support structure 110 may produce a radially inward force on the mandrel 360 and/or the layer 370 of nonwoven fibres. In other words, the support structure 110, in the second diameter configuration on the mandrel 360, may squeeze the mandrel and/or the layer 370 of nonwoven fibres. Such an inward force may aid in embedding the support structure 110 within the layer 370 of nonwoven fibres.

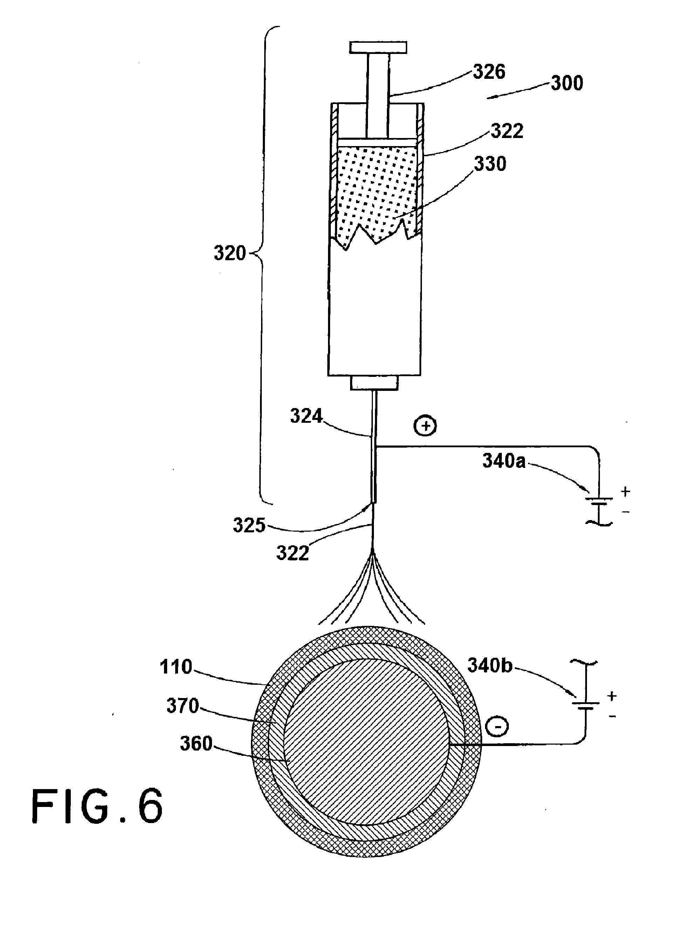

A layer of nonwoven fibres may be formed on the abluminal surface 118 of the support structure 110 by electrospinning the solution 330 from the orifice 324 onto the abluminal surface of the support structure. **FIG. 6** illustrates the electrospinning apparatus 300 described above configured to form a layer of nonwoven fibres on the abluminal surface 118 of the support structure 110. The support structure 110 may be positioned between the spinneret 320 and the mandrel 360. The solution 330 may be discharged from the spinneret 320 toward the mandrel 360 as described above. The stream 322 may contact the abluminal surface 118 of the support structure 110 to form the layer of nonwoven electrospun fibres on the abluminal surface.

In one example, the mandrel 360 may be moved rotationally about the longitudinal axis thereof, which may cause corresponding rotation of the support structure 110. The solution 330 may be discharged from the orifice 324 and attracted to the mandrel 360 by the electrical potential applied between the orifice and the mandrel as described above. The rotation of the support structure 110 may cause the resultant coating of nonwoven fibres to be distributed about the circumference of the support structure. Additionally, or alternatively, the spinneret 320 may be translated longitudinally relative to the support structure 110 while discharging the solution 330 from the orifice 324. The translation of the spinneret 320 may cause the resultant coating of nonwoven fibres to be distributed about the length of the support structure 110. In one example, the support structure 110 may be rotated and the spinneret 320 may be translated to form a layer of nonwoven fibres covering substantially the entire circumference of the support structure along at least a portion of the length of the support structure.

In one example, the support structure 110 may be electrically charged during electrospinning of the layer of nonwoven fibres on the abluminal surface thereof. In other words, an electrical potential may be applied between the orifice and the support structure 110. The electrical potential may aid in attracting the solution 330 discharged from the orifice 324 as described above. The electrical charge on the support structure 110 may be generated, for example, by the electrical charge on the mandrel 360 and the proximity of the support structure to the mandrel. Additionally, or alternatively, the support structure 110 may be electrically coupled to the mandrel 360 (e.g., with a conductive wire or by contact with the mandrel). The electrical charge on the support structure 110 and/or the mandrel 360 may vary during electrospinning. For example, the electrical charge may be reduced by an insulating effect of the layers of electrospun fibres formed on the mandrel 360 and/or the support structure 110. The electrical charges of the mandrel 360, the support structure 110, and/or the spinneret 320 may be adjusted (e.g., increased) during electrospinning to compensate for such an insulating effect.

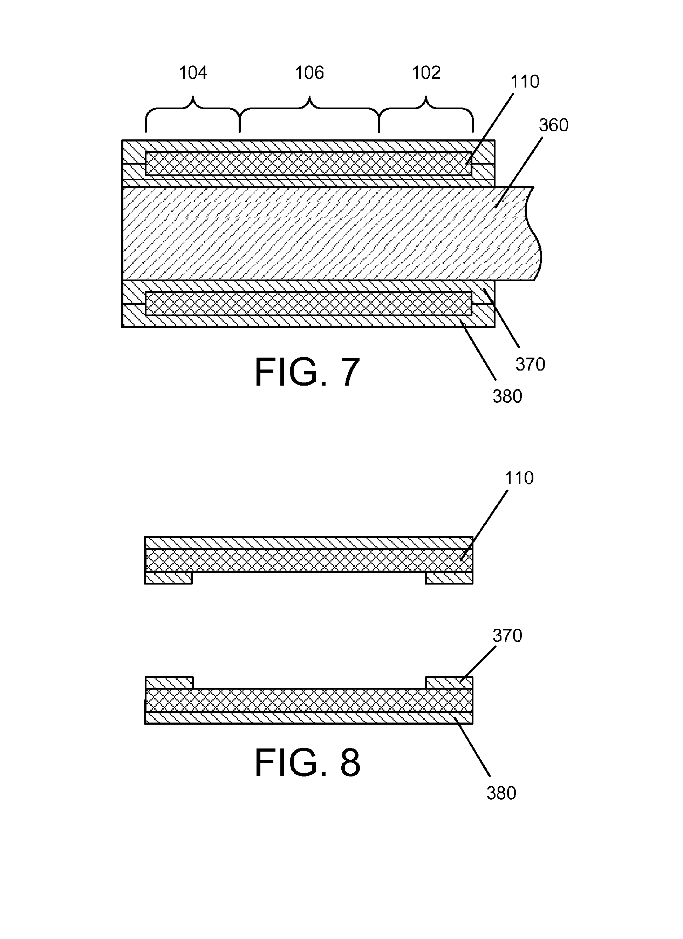

**FIG. 7** shows a longitudinal cross sectional view of the support structure 110 positioned on the mandrel 360 between the layer 370 of nonwoven electrospun fibres disposed on the outer surface of the mandrel and a layer 380 of nonwoven electrospun fibres disposed on the abluminal surface 118 of the support structure. The support structure 110 may include a proximal end segment 102, a distal end segment 104, and an intermediate segment 106 positioned between the proximal end segment and the distal end segment. The proximal end segment 102 of the support structure 110 may be positioned adjacent to the proximal end segment 364 of the mandrel 360; the distal end segment 104 of the support structure may be positioned adjacent to the distal end segment 366 of the mandrel; and/or the intermediate segment 106 of the support structure may be positioned adjacent to the intermediate segment 368 of the mandrel. In other words, each segment of the support structure 110 may be at least partially aligned with the corresponding segment of the mandrel 360 as shown in **FIG. 7**. The support structure 110 may be rotated and the spinneret 320 may be translated while the solution 330 is electrospun onto the abluminal surface 118 of the support structure as described above to distribute the electrospun fibres circumferentially and longitudinally about the abluminal surface of the support structure. In one example, the solution 330 may be electrospun onto each of the proximal end segment 102, the distal end segment 104, and the intermediate segment 106 of the support structure 110 such that the layer 380 may be disposed upon substantially the entire length of the abluminal surface 118 of the support structure as shown in **FIG. 7**. In other words, each of the proximal end segment 102, the distal end segment 104, and the intermediate segment 106 of the support structure 110 may be coated with the electrospun fibres. Portions of the layer 370 of nonwoven electrospun fibres or the layer 380 of nonwoven electrospun fibres may be omitted during electrospinning or removed from the support structure 110 and/or the mandrel 360 subsequent to electrospinning to form a partially encapsulated support structure as further described below.

Upon forming the layer 380 of electrospun fibres on the abluminal surface 118 of the support structure 110, the support structure may be at least partially encapsulated within a covering formed by the layer 370 of nonwoven fibres and the layer 380 of nonwoven fibres as shown in **FIG. 7**. In other words, the support structure 110 may be laminated between luminal and abluminal layers of graft material. The layer 370 and the layer 380 may be joined to one another to form the covering around the support structure 110. For example, the layer 370 and the layer 380 may contact one another through the openings formed between the ring structures 112 and the connector segments 114 of the support structure 110. The portions of the layer 370 and the layer 380 in contact with one another may bond to one another to join the layer 370 and the layer 380 to one another. In one example, the support structure 110 may be embedded in the layer 380 of nonwoven fibres as described above with reference to the layer 370 such that the layer 380 and the layer 370 may contact one another through the openings of the support structure. The layer 370 and the layer 380 may be joined to one another during the electrospinning process without an additional bonding process (e.g., heat or pressure bonding) and/or without additional bonding materials (e.g., adhesives, sutures, staples, or clips). The layer 370 and the layer 380 may be disposed on substantially the entire length of the support structure 110 as shown in **FIG. 7** to encapsulate substantially the entire support structure between the layer 370 and the layer 380 of electrospun fibres. Portions of the layer 370 or the layer 380 may be omitted or removed to encapsulate a portion of the support structure 110 between the layer 370 and the layer 380 of electrospun fibres while leaving a portion of the support structure unencapsulated as further described below. The covering formed by the layer 370 and the layer 380 of electrospun fibres may form the graft body 120 of the prosthesis 100. The support structure 110 and the covering (i.e., the graft body 120) may collectively form the prosthesis 100 (e.g., a covered stent).

The prosthesis 100 (e.g., the support structure 110 with the layer 370 and the layer 380 of electrospun fibres attached thereto) may be removed from the mandrel 360. To that end, the mandrel 360 may include a release layer applied to the outer surface thereof. The release layer may reduce the attractive force (e.g., adhesive force) or the frictional force between the layer 370 of electrospun fibres disposed on the luminal surface of the support structure 110 and the outer surface of the mandrel 360 to aid in removing the prosthesis 100 from the mandrel in an undamaged condition. Upon removal from the mandrel 360, the support structure 110 may contract to the relaxed diameter. Forming the layer 370 and the layer 380 of electrospun fibres on the support structure 110 in the over-expanded configuration may enhance the flexibility of the prosthesis 100. For example, upon contraction of the support structure 110 to the relaxed diameter, the tension of the layer 370 and/or the layer 380 of electrospun fibres may be reduced, which may enable increased movement or flexibility of the covering.

An excess length of the covering formed by the layer 370 and the layer 380 of electrospun fibres may extend beyond at least one of the proximal end segment or the distal end segment of the support structure 110 as shown in **FIG. 7**. At least a portion of the excess length of the covering may be removed from the prosthesis 100. In other words, the excess covering material extending beyond one or more of the ends of the support structure 110 may be trimmed from the prosthesis 100 such that the ends of the covering may be substantially aligned with the ends of the support structure.

The thickness of the graft body 120 of the prosthesis 100 may be manipulated by the electrospinning operator (e.g., by adjusting the speed of rotation and/or translation) for desired operational use. Additionally, or alternatively, the fully or partially encapsulated electrospun covered support structure (e.g., the covered stent) may be post-processed using manufacturing techniques (e.g., laser welding/marking, mechanical punching, etc.) to create varying porosity if desired. Additionally, or alternatively, the graft or covering material may be electrospun simultaneously with additional polymers, additives, or pharmacological agents to promote mechanical and/or chemical characteristics of the prosthesis. For example, the graft or covering material may be electrospun simultaneously with additional polymers such as PET (e.g., DACRON®, commercially available from Invista, Wichita, KS) or polyurethane (e.g., THORALON®, commercially available from Thoratec, Pleasanton, CA). In one example, the graft or covering material may include PET and polyurethane. Additionally, or alternatively, the graft or covering material may be electrospun simultaneously with additives or pharmacological agents such as, for example, lauric acid, levulinic acid, or polyethylene glycol (e.g., having a molecular weight of about 300, about 600, or any other suitable molecular weight). Electrospinning the graft or covering material with other materials may affect the mechanical properties (e.g., flexibility or strength) of the graft or covering material. Additionally, or alternatively, electrospinning the graft or covering material with other materials may affect the frictional properties and/or enable a reduced profile of the graft or covering material.

The support structure 110 may be substantially entirely or partially encapsulated within the covering to form the prosthesis 100. In other words, the prosthesis may be configured as a fully or partially encapsulated stent. **FIG. 8** illustrates one example of the support structure 110 partially encapsulated within the covering to form a partially encapsulated stent. The layer 370 of nonwoven fibres may be disposed upon only a portion of the length of the luminal surface 117 of the support structure 110. This may be achieved by forming the layer 370 of nonwoven fibres on a portion of the working length of the mandrel 360 during preparation of the prosthesis. In one example, substantially the entire working length of the mandrel 360 may be coated with the layer 370 of nonwoven fibres as described above. A portion of the layer 370 may be selectively removed from the mandrel. For example, a central portion of the layer 370 disposed on the intermediate segment 368 of the mandrel 360 may be removed. The portion of the layer 370 may be removed from the mandrel in any suitable manner such as, for example, severing the layer 370 at the ends of the intermediate segment 368 (e.g., between the intermediate segment and each of the proximal end segment 364 and the distal end segment 366) and lifting the nonwoven fibres from the mandrel.

In another example, the layer 370 may be formed on a portion of the working length of the mandrel 360 by controlling the movement of the spinneret 320 and the mandrel 360 relative to one another during the electrospinning process as described above. For example, the longitudinal translation of the spinneret 320 may be limited such that substantially no fibres are electrospun onto the intermediate segment 368 of the mandrel. Additionally, or alternatively, the flow of the solution 330 from the orifice 325 may be interrupted (e.g., stopped and restarted) during the electrospinning process to avoid electrospinning fibres onto the intermediate segment 368 of the mandrel 360. In other words, a portion of the layer 370 may be electrospun on the proximal end segment 364 of the mandrel 360, and another portion of the layer 370 may be electrospun on the distal end segment 366. The flow of the solution 330 may be interrupted as the spinneret moves across the intermediate segment 368 of the mandrel 360 so that substantially no fibres are electrospun onto the intermediate segment.

In yet another example, the layer 370 may be formed on a portion of the working length of the mandrel 360 by shielding a portion of the mandrel during the electrospinning process. For example, a portion of the mandrel (e.g., the intermediate segment 368) may be covered with a shielding material (e.g., a removable non-adhesive material, such as TEFLON® tubing, or a band of insulating tape) so that the covered portion of the mandrel remains uncoated during the electrospinning process. In still another example, a diffusion template may be used to shield a portion of the mandrel. For example, the diffusion template may be configured as a plate including an opening formed therein. The plate may be placed between the spinneret 320 and the mandrel 360 to obstruct the electrospun fibres from reaching a determined portion (e.g., the intermediate segment 368) of the mandrel.

The support structure 110 may be placed on the mandrel 360 over the layer 370 of nonwoven fibres with the intermediate segment 106 of the support structure substantially aligned with the uncovered intermediate segment 368 of the mandrel. The layer 380 of nonwoven fibres may be formed on the abluminal surface 118 of the support structure as described above. In this manner, a prosthesis having an abluminal covering with luminal end encapsulation at each of the proximal and distal ends may be formed. The proximal end segment 102 and the distal end segment 104 of the support structure 110 may be encapsulated within the covering formed by the layer 370 and the layer 380 of nonwoven electrospun fibres. The graft material covering may be bonded to the support structure 110 by the end encapsulation of the proximal end segment 102 and the distal end segment 104. Such end encapsulation may provide a strong adhesion between the graft material and the support structure at the ends of the prosthesis. The intermediate segment 106 of the luminal surface 117 of the support structure 110 may be uncovered. In other words, the intermediate segment of the support structure may be unencapsulated.

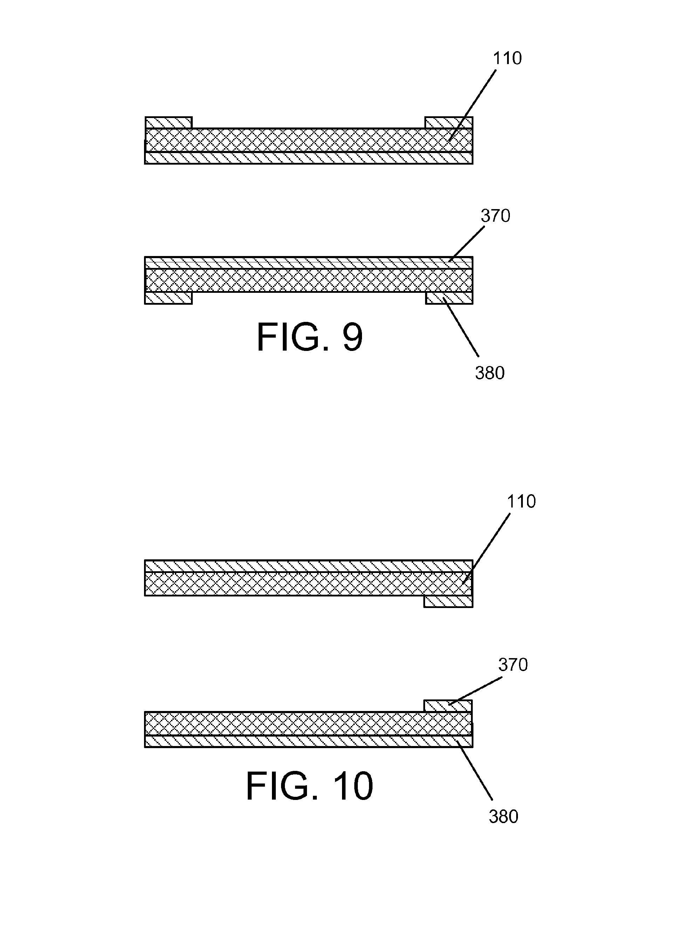

**FIG. 9** illustrates another example of the support structure 110 partially encapsulated within the covering to form a partially encapsulated stent. The layer 370 of nonwoven fibres may be disposed upon substantially the entire length of the luminal surface 117 of the support structure 110 by electrospinning the solution 330 onto substantially the entire working length of the mandrel 360 as described above. The layer 380 of nonwoven fibres may be disposed upon only a portion of the length of the support structure 110. In one example, substantially the entire length of the support structure 110 may be coated with the layer 380 of nonwoven fibres as described above. A portion of the layer 380 may be removed from the support structure 110. For example, a central portion of the layer 380 disposed on the intermediate segment 106 of the support structure 110 may be removed. The portion of the layer 380 may be removed from the support structure 110 in any suitable manner as described above with reference to removing a portion of the layer 370 from the mandrel 360. In another example, the layer 380 may be formed on a portion of the length of the support structure 110 by controlling the movement of the 21 spinneret 320 and the mandrel 360 relative to one another during the electrospinning process as described above. For example, the longitudinal translation of the spinneret 320 may be limited and/or the flow of solution 330 may be interrupted such that substantially no fibres are electrospun onto the intermediate segment 106 of the support structure 110. In yet another example, the layer 380 may be formed on a portion of the length of the support structure 110 by shielding a portion of the support structure during the electrospinning process as described above. For example, a portion of the support structure (e.g., the intermediate segment 106) may be covered with a length of removable, non-adhesive material (e.g., TEFLON® tubing) which may keep material off of a portion of the abluminal surface (e.g., the central abluminal surface) so that the covered portion of the support structure remains uncoated during the electrospinning process.

In this manner, a prosthesis having a luminal covering with abluminal end encapsulation at each of the proximal and distal ends may be formed. The proximal end segment 102 and the distal end segment 104 of the support structure 110 may be encapsulated within the covering formed by the layer 370 and the layer 380 of nonwoven electrospun fibres, and the intermediate segment 106 of the abluminal surface 117 of the support structure may be uncovered. In other words, the intermediate segment of the support structure may be unencapsulated.

**FIG. 10** illustrates another example of the support structure 110 partially encapsulated within the covering to form a partially encapsulated stent. The prosthesis may include an abluminal covering with luminal end encapsulation at only one of the proximal end or the distal end of the prosthesis. This may be achieved by forming the layer 370 on a only a portion of the length of the luminal surface 117 of the support structure 110 as described above and forming the layer 380 on substantially the entire length of the abluminal surface 118 of the support structure also as described above. One of the proximal end segment 102 or the distal end segment 104 of the support structure 110 may be encapsulated within the covering formed by the layer 370 and the layer 380 of nonwoven electrospun fibres, and the other of the proximal end segment 102 or the distal end segment 104 may be uncovered along the luminal surface thereof. The intermediate segment 106 of the luminal surface 117 of the support structure may be uncovered. In other words, one of the proximal end segment or the distal end segment and the intermediate segment of the support structure may be unencapsulated. Although **FIG. 10** illustrates a prosthesis including an abluminal covering with proximal end encapsulation, a prosthesis having an abluminal covering with distal end encapsulation or a luminal covering with proximal end encapsulation or distal end encapsulation may be formed in a similar manner.

A covering formed by electrospinning as described herein may include a plurality of nonwoven fibres. In other words, the electrospun fibres may be configured as a mesh of fibres as opposed to a patterned weave or knit of fibres. The electrospun fibres may be nanofibers having a diameter of less than about 1,000 nm. Additionally, or alternatively, the electrospun covering may substantially conform to the underlying support structure. In other words, the electrospun covering may substantially take the shape of the support structure and may be substantially free of ridges or puckering which may be caused by mechanical attachment mechanisms (e.g., sutures). Additionally, or alternatively, the electrospun covering may be substantially seamless. In other words, the covering may be substantially free of seams which may be formed, for example, by stitching together or otherwise attaching adjacent edges of one or more sheets of graft material.

In other examples, a partially encapsulated support structure may be formed using any other suitable method including, for example, dip coating, spray coating, and melt-spinning. For example, a layer of graft material may be deposited on the outer surface of a mandrel by dipping the mandrel into a volume of liquid graft material. The liquid graft material on the outer surface of the mandrel may be dried or cured to form a layer of graft material on the mandrel. A support structure may be placed over the layer of graft material formed on the mandrel, and an end portion of the mandrel and the support structure may be dipped into the volume of liquid graft material to deposit a layer of graft material on the abluminal surface of the support structure. In this manner, the end portion of the support structure may be encapsulated between the two layers of graft material. The opposite end portion of the mandrel and the support structure may be dipped into the volume of liquid graft material to encapsulate the other end portion if desired. A partially encapsulated support structure may be formed in a similar manner using any other suitable method to deposit a layer of graft material on a surface of the mandrel and/or the support structure.

Applying a covering material to a support structure by electrospinning and/or encapsulating only a portion of the support structure as described herein may enable formation of a prosthesis having a reduced profile. For example, the covering may be attached to the support structure without the use of any attachment material (e.g., suture, tape, such as PTFE-FEP bonding tape, glue, or lamination material) or additional processing steps (e.g., mechanical attachment, pressure bonding, chemical treatment, or thermal bonding). In other words, the prosthesis may be substantially free of an extrinsic attachment mechanism. Such attachment material may increase the thickness of the coating resulting in a prosthesis having a larger profile. Additionally, or alternatively, partial encapsulation may provide a prosthesis having a reduced amount of covering material compared to a fully encapsulated support structure, which may result in a reduced profile. In one example, the thickness of a portion of the graft body (e.g., a portion of the covering corresponding or positioned adjacent to the intermediate segment of the support structure) may have a thickness of less than about 70 µm, preferably less than about 25 µm. A graft body with a reduced thickness may enable a prosthesis having a reduced profile. In other words, reducing the thickness of the graft body may enable reduction of the profile (e.g., the thickness or the diameter) of the prosthesis. Such a low-profile prosthesis may be delivered using a sheath having a reduced profile relative to conventional introducer sheaths. This may aid in advancing the sheath within a body vessel to the delivery site within the patient's anatomy.

Partial encapsulation of a support structure may enable a prosthesis having increased flexibility compared to a fully encapsulated support structure. For example, a low-profile prosthesis may have increased flexibility resulting from, for example, lack of attachment materials and/or reduced thickness. Increased flexibility may enable increased manoeuvrability to deliver the prosthesis through tortuous anatomy. Leaving portions of the support structure uncovered (i.e. partial encapsulation of the support structure) may enable at least a portion of the graft body to slide relative to the support structure. For example, as the prosthesis is compressed (e.g., to be loaded into a delivery device), the length of the support structure may increase. One end of the graft body may be unattached to the support structure. The support structure may be capable of moving relative to the unattached portion of the graft body. For example, the unattached portion of the graft body may be capable of sliding relative to the support structure so that the length of the support structure relative to the graft body may change. In one example, the length of the graft body may remain substantially constant as the length of the support structure changes. This may reduce the potential for stretching or damaging (e.g., by ripping or tearing) the graft material during contraction and/or expansion of the prosthesis.

Direct encapsulation of the support structure may reduce abrasive forces between the graft material and the support structure. For example, the portion of the support structure encapsulated within the graft material may be substantially unable to move relative to the graft material, thereby reducing abrasion between the encapsulated support structure and the graft material. Additionally, or alternatively, direct encapsulation of the support structure may enable formation of a substantially non-porous graft material that is substantially free of suture holes or other openings formed therein.

Solutions for use in the electrospinning process of the present disclosure may include any suitable liquids containing materials to be electrospun (e.g., any of the graft materials described above). For example, solutions may include, but are not limited to, suspensions, emulsions, melts, and hydrated gels containing the materials, substances, or compounds to be electrospun. Solutions also may include solvents or other liquids or carrier molecules. Solutions may include, for example, any of the materials described in U.S. Patent No. 7,799,261 to Orr et al.*,* which is incorporated herein by reference. In one example, the solution 330 may include a PET such as, for example a DACRON® leg-fabric commercially available from Invista, Wichita, KS. The solution 330 may include a polymer solution of PET in approximately 50:50 trifluoroacetic acid (TFA) and dichloromethane (DCM or methylene chloride) at a predetermined concentration, typically between about 0.10 g/mL and about 0.17 g/mL solvent.

Additionally, or alternatively, solutions may include one or more bioactive agents. A therapeutically effective amount of a bioactive agent may be incorporated into the graft material produced by the electrospinning process for implantation within a patient. The bioactive agent may be selected to perform a desired function upon implantation. For example, the bioactive agent may be selected to treat indications such as atherosclerosis, renal dialysis fistulae stenosis, or vascular graft stenosis. A graft material including a bioactive agent may be useful when performing procedures such as coronary artery angioplasty, renal artery angioplasty, or carotid artery surgery. Also for example, a bioactive agent such as a growth factor may be selected to promote ingrowth of tissue from the interior wall of a body vessel. An anti-angiogenic or antineoplastic bioactive agent such as paclitaxel, sirolimus, or a rapamycin analogue, or a metalloproteinase inhibitor such as batimastaat may be included to mitigate or prevent undesired conditions in the vessel wall, such as restenosis. Many other types of bioactive agents also may be included in the solution.

Although the electrospinning process has been described in relation to applying a graft material covering to a support structure, this disclosure is not so limited. The electrospinning process described above may be used to apply any type of coating to any type of medical device. For example, the electrospinning process may be used to apply a coating of a therapeutic agent to a stent or a covered stent (e.g., a stent graft).

In one example, a method for preparing an endoluminal prosthesis may include providing a support structure including a proximal end segment, a distal end segment, a lumen extending longitudinally within the support structure, a luminal surface, and an abluminal surface opposite the luminal surface. The method may include providing an electrospinning apparatus including an orifice and a mandrel. An electric potential may be applied between the orifice and the mandrel. A first layer of nonwoven fibres may be formed on an outer surface of the mandrel by electrospinning a solution from the orifice onto the outer surface of the mandrel. The first layer of nonwoven fibres may be contacted with the luminal surface of the support structure by locating the mandrel at least partially within the lumen of the support structure. A second layer of nonwoven fibres may be formed on the abluminal surface of the support structure by electrospinning the solution from the orifice onto the abluminal surface of the support structure. The support structure may be partially encapsulated within a covering formed by the first layer of nonwoven fibres and the second layer of nonwoven fibres so that at least a portion of the support structure is unencapsulated within the covering.

An excess length of the covering may extend beyond at least one of the proximal end segment or the distal end segment of the support structure, and the method may include trimming at least a portion of the excess length of the covering from the partially encapsulated support structure. A first electrical charge may be applied to the orifice, and a second electrical charge may be applied to the mandrel. The second electrical charge may be opposite of the first electrical charge. Electrospinning the solution from the orifice onto the outer surface of the mandrel may include electrospinning a first solution from the orifice onto the outer surface of the mandrel, electrospinning the solution from the orifice onto the abluminal surface of the support structure may include electrospinning a second solution from the orifice onto the abluminal surface of the support structure, and the first solution may be different from the second solution.

The support structure may include an intermediate segment positioned between the proximal end segment and the distal end segment of the support structure, and the outer surface of the mandrel may include a first end segment corresponding to the proximal end segment of the support structure, a second end segment corresponding to the distal end segment of the support structure, and an intermediate segment corresponding to the intermediate segment of the support structure. The first layer of nonwoven fibres may cover at least one of the first end segment or the second end segment of the outer surface of the mandrel, the intermediate segment of the outer surface of the mandrel may be substantially uncovered by the first layer of nonwoven fibres, and the second layer of nonwoven fibres may cover each of the proximal end segment, the distal end segment, and the intermediate segment of the abluminal surface of the support structure. At least one of the proximal end segment or the distal end segment of the support structure corresponding to the at least one of the first end segment or the second end segment of the outer surface of the mandrel may be encapsulated within the covering formed by the first layer of nonwoven fibres and the second layer of nonwoven fibres, and the intermediate segment may be unencapsulated within the covering. Forming the first layer of nonwoven fibres on the outer surface of the mandrel may include electrospinning the solution from the orifice onto the intermediate segment of the outer surface of the mandrel and uncovering the intermediate segment of the outer surface of the mandrel by removing a central portion of the first layer of nonwoven fibres disposed on the intermediate segment of the outer surface of the mandrel. The first layer of nonwoven fibres may cover each of the first end segment, the second end segment, and the intermediate segment of the outer surface of the mandrel, the second layer of nonwoven fibres may cover at least one of the proximal end segment or the distal end segment of the abluminal surface of the support structure, and the intermediate segment of the abluminal surface of the support structure may be substantially uncovered by the second layer of nonwoven fibres. The at least one of the proximal end segment or the distal end segment of the support structure may be encapsulated within the covering formed by the first layer of nonwoven fibres and the second layer of nonwoven fibres, and the intermediate segment may be unencapsulated within the covering.

Contacting the first layer of nonwoven fibres with the luminal surface of the support structure may include over-expanding the support structure to a first over-expanded diameter, and allowing the support structure to contract to a second over-expanded diameter over the first layer of nonwoven fibres. The first over-expanded diameter may be larger than the second over-expanded diameter, and the second over-expanded diameter may be larger than a relaxed diameter of the support structure.

In one example, a method for preparing an endoluminal prosthesis may include providing a support structure including a first end segment, a second end segment, an intermediate segment positioned between the first end segment and the second end segment of the support structure, a lumen extending longitudinally within the support structure, a luminal surface, and an abluminal surface opposite the luminal surface. The method may include providing an electrospinning apparatus including an orifice and a mandrel. The mandrel may include a first end segment, a second end segment, and an intermediate segment positioned between the first end segment and the second end segment of the mandrel. A first electrical charge may be applied to the orifice. A second electrical charge may be applied to the mandrel. A first layer of nonwoven fibres may be formed on an outer surface of the mandrel by electrospinning a solution from the orifice onto the first end segment of the mandrel. The mandrel may be located at least partially within the lumen of the support structure. The first end segment of the support structure may be aligned with the first end segment of the mandrel, the second end segment of the support structure may be aligned with the second end segment of the mandrel, and the intermediate segment of the support structure may be aligned with the intermediate segment of the mandrel. The first layer of nonwoven fibres may be contacted with the luminal surface of the support structure. The first layer of nonwoven fibres may cover the luminal surface of the first end segment of the support structure. A second layer of nonwoven fibres may be formed on the abluminal surface of the support structure by electrospinning the solution from the orifice onto the abluminal surface of the first end segment of the support structure. The second layer of nonwoven fibres may cover the abluminal surface of the first end segment of the support structure. The first end segment of the support structure may be encapsulated within a covering formed by the first layer of nonwoven fibres and the second layer of nonwoven fibres. One of the luminal surface or the abluminal surface of at least one of the intermediate segment or the second end segment of the support structure may be uncovered by the respective first layer of nonwoven fibres or second layer of nonwoven fibres.

Forming a first layer of nonwoven fibres on an outer surface of the mandrel may include electrospinning the solution from the orifice onto the second end segment of the mandrel. The first layer of nonwoven fibres may cover the luminal surface of the second end segment of the support structure. Forming a second layer of nonwoven fibres on the abluminal surface of the support structure may include electrospinning the solution from the orifice onto the abluminal surface of the second end segment of the support structure. The second layer of nonwoven fibres may cover the abluminal surface of the second end segment of the support structure. Both the first end segment and the second end segment of the support structure may be encapsulated within the covering formed by the first layer of nonwoven fibres and the second layer of nonwoven fibres.

Forming a first layer of nonwoven fibres on an outer surface of the mandrel may include electrospinning the solution from the orifice onto the intermediate segment of the mandrel. The first layer of nonwoven fibres may cover the luminal surface of the intermediate segment of the support structure. The abluminal surface of the intermediate segment of the support structure may be uncovered by the second layer of nonwoven fibres. The intermediate segment of the support structure may be unencapsulated within the covering formed by the first layer of nonwoven fibres and the second layer of nonwoven fibres.

The luminal surface of the intermediate segment of the support structure may be uncovered by the first layer of nonwoven fibres. Forming a second layer of nonwoven fibres on the abluminal surface of the support structure may include electrospinning the solution from the orifice onto the abluminal surface of the intermediate segment of the support structure. The second layer of nonwoven fibres may cover the abluminal surface of the intermediate segment of the support structure. The intermediate segment of the support structure may be unencapsulated within the covering formed by the first layer of nonwoven fibres and the second layer of nonwoven fibres.

Forming a first layer of nonwoven fibres on an outer surface of the mandrel may include electrospinning the solution from the orifice onto each of the first end segment, the second end segment, and the intermediate segment of the mandrel, and selectively removing a portion of the nonwoven electrospun fibres from at least one of the second end segment or the intermediate segment of the mandrel.

While various embodiments of the invention have been described, the invention is not to be restricted except in light of the attached claims and their equivalents. Moreover, the advantages described herein are not necessarily the only advantages of the invention and it is not necessarily expected that every embodiment of the invention will achieve all of the advantages described.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 13/618,356, from which this application claims priority, and in the abstract accompanying this application are incorporated herein by reference.

## Claims

1. An endoluminal prosthesis for placement within a body vessel, the prosthesis including:
a tubular support structure including a proximal end segment, a distal end segment, an intermediate segment positioned between the proximal end segment and the distal end segment, a lumen extending longitudinally within the support structure, a luminal surface, and an abluminal surface opposite the luminal surface;
a first layer of nonwoven electrospun fibres positioned on the luminal surface of the support structure; and
a second layer of nonwoven electrospun fibres positioned on the abluminal surface of the support structure;
wherein at least one of the proximal end segment or the distal end segment of the support structure is encapsulated within a covering comprising the first layer of nonwoven electrospun fibres and the second layer of nonwoven electrospun fibres, and the intermediate segment of the support structure is unencapsulated within the covering.

2. A prosthesis according to claim 1, wherein:
the luminal surface of the at least one of the proximal end segment or the distal end segment of the support structure is covered by the first layer of nonwoven electrospun fibres, the abluminal surface of the at least one of the proximal end segment or the distal end segment of the support structure is covered by the second layer of nonwoven electrospun fibres, and the at least one of the proximal end segment or the distal end segment of the support structure is encapsulated within the covering: or
the luminal surface of each of the proximal end segment and the distal end segment of the support structure is covered by the first layer of nonwoven electrospun fibres, the abluminal surface of each of the proximal end segment and the distal end segment of the support structure is covered by the second layer of nonwoven electrospun fibres, and each of the proximal end segment and the distal end segment is encapsulated within the covering; or
one of the luminal surface or the abluminal surface of the intermediate segment of the support structure is covered by the respective first layer or second layer of nonwoven electrospun fibres, and the other of the luminal surface or the abluminal surface of the intermediate segment of the support structure is uncovered by the respective first layer or second layer of nonwoven electrospun fibres.

3. A prosthesis according to any preceding claim, wherein an intermediate portion of the covering corresponding to the intermediate segment of the support structure comprises a thickness of between about 10 µm and about 70 µm or of between about 10 µm and about 25 µm.

4. A prosthesis according to any preceding claim, wherein the first layer of nonwoven electrospun fibres and the second layer of nonwoven electrospun fibres are attached to the support structure without an extrinsic attachment mechanism.

5. A prosthesis according to any preceding claim, wherein a portion of the first layer of nonwoven electrospun fibres is in contact with a portion of the second layer of nonwoven electrospun fibres through an opening in the support structure.

6. A prosthesis according to claim 5, wherein the portion of the first layer of nonwoven electrospun fibres and the portion of the second layer of nonwoven electrospun fibres are bonded to one another without an extrinsic bonding material.

7. A prosthesis according to any preceding claim, wherein the unencapsulated intermediate segment of the support structure is movable relative to the covering.

8. An endoluminal prosthesis including:
a support structure comprising a proximal end segment, a distal end segment, a lumen extending longitudinally within the support structure, a luminal surface, and an abluminal surface opposite the luminal surface;
a first layer of nonwoven electrospun fibres disposed on the luminal surface of the support structure; and
a second layer of nonwoven electrospun fibres disposed on the abluminal surface of the support structure;
wherein the first layer of nonwoven electrospun fibres and the second layer of nonwoven electrospun fibres cooperatively form a covering, the support structure is partially encapsulated within the covering, and at least a portion of the support structure is unencapsulated within the covering.

9. A prosthesis according to claim 8, wherein the unencapsulated portion of the support structure is unattached to the covering.

10. A prosthesis according to claim 8 or 9, wherein at least one of the proximal end segment or the distal end segment of the support structure is encapsulated within the covering.

11. A prosthesis according to claim 10, wherein the covering is attached to the support structure at the encapsulated proximal end segment or distal end segment, and the prosthesis is free of any extrinsic attachment mechanism between the covering and the support structure.

12. A prosthesis according to claim 10 or 11, wherein the support structure includes an intermediate segment positioned between the proximal end segment and the distal end segment, and the intermediate segment of the support structure is unencapsulated within the covering.

13. A prosthesis according to claim 12, wherein:
the luminal surface of each of the proximal end segment, the intermediate segment, and the distal end segment of the support structure is covered by the first layer of nonwoven electrospun fibres, and the abluminal surface of the intermediate segment of the support structure is uncovered by the second layer of nonwoven electrospun fibres; and/or
the luminal surface of the intermediate segment of the support structure is uncovered by the first layer of nonwoven electrospun fibres, and the abluminal surface of each of the proximal end segment, the intermediate segment, and the distal end segment of the support structure is covered by the second layer of nonwoven electrospun fibres.

14. A prosthesis according to any preceding claim, wherein the covering includes polyethylene terephthalate and polyurethane.

15. A prosthesis according to any preceding claim, wherein the covering includes a pharmacological agent selected from the group consisting of lauric acid, levulinic acid, polyethylene glycol, and combinations thereof.

16. A method of preparing an endoluminal prosthesis, including the steps of:
providing a support structure comprising a proximal end segment, a distal end segment, a lumen extending longitudinally within the support structure, a luminal surface, and an abluminal surface opposite the luminal surface;
providing an electrospinning apparatus including an orifice and a mandrel;
applying an electric potential between the orifice and the mandrel;
forming a first layer of nonwoven fibres on an outer surface of the mandrel by electrospinning a solution from the orifice onto the outer surface of the mandrel;
contacting the first layer of nonwoven fibres with the luminal surface of the support structure by locating the mandrel at least partially within the lumen of the support structure; and
forming a second layer of nonwoven fibres on the abluminal surface of the support structure by electrospinning the solution from the orifice onto the abluminal surface of the support structure;
whereby the support structure is partially encapsulated within a covering formed by the first layer of nonwoven fibres and the second layer of nonwoven fibres so that at least a portion of the support structure is unencapsulated within the covering.
